(19) Europäisches Patentamt
European Patent Office
Office européen des brevets

(11) EP 1 674 581 A1

(12) **EUROPEAN PATENT APPLICATION**

(43) Date of publication:
**28.06.2006 Bulletin 2006/26**

(51) Int Cl.:
***C12Q 1/68*** *(2006.01)* ***C07H 21/00*** *(2006.01)*

(21) Application number: **04030341.4**

(22) Date of filing: **21.12.2004**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Max-Planck-Gesellschaft zur Förderung der Wissenschaften e.V.**
**Berlin (DE)**

(72) Inventors:
- **Müllen, Klaus**
**50939 Köln (DE)**
- **Herrmann, Andreas**
**65199 Wiesbaden (DE)**
- **Ergen, Erhan**
**55131 Mainz (DE)**

(74) Representative: **Vossius & Partner**
**Siebertstrasse 4**
**81675 München (DE)**

(54) **Twin probes, a tool for the detection of SNPs**

(57) This invention relates to a compound having the general formula (I) ON1 - L1 - F - L2 - ON2 wherein ON1 and ON2 are independently selected from the group consisting of oligodeoxyribonucleotides, oligoribonucleotides or PNAs; L1 and L2 are linkers; and F is a fluorophore. Furthermore provided are a method of detecting a target polynucleotide in a sample, a method of detecting one or more mutations or single nucleotide polymorphisms in a target polynucleotide in a sample, a method for the simultaneous detection of a plurality of alleles of a target polynucleotide in a sample as well as kits for performing these methods.

EP 1 674 581 A1

## Description

This invention relates to a compound having the general formula (I) ON1 - L1 - F - L2 - ON2 wherein ON1 and ON2 are independently selected from the group consisting of oligodeoxyribonucleotides, oligoribonucleotides or PNAs; L1 and L2 are linkers; and F is a fluorophore. Furthermore provided are a method of detecting a target polynucleotide in a sample, a method of detecting one or more mutations or single nucleotide polymorphisms in a target polynucleotide in a sample, a method for the simultaneous detection of a plurality of alleles of a target polynucleotide in a sample as well as kits for performing these methods.

In this specification, a number of documents is cited. The disclosure of these documents, including manufacturer's manuals, is herewith incorporated by reference in its entirety.

With the completion of the human genome project, maps of human sequence variations have been reported and deposited in public databases.[1-3] Among these variations, single nucleotide polymorphisms (SNPs) are the most frequently ones. According to The International SNP Map Working Group the number of SNPs in the human genome is approximately 1.42 million, 60.000 of them being located in exons.[4] SNPs in the coding region are of particular interest as they are more likely to change the biological function of a protein. In the future, SNP detection is assumed to play an important role in determining a person's genetic predisposition for inherited diseases, disease diagnosis, and personalized medicine.[5] Therefore, there is a strong interest to develop rapid, sensitive, and cheap methods for SNP detection. At present various methods are available for SNP detection which have recently been summarized.[6, 7] Many of these methods are based on fluorescence detection, especially when parallel assays and high throughput screening are envisaged. For the identification of SNPs by hybridization in homogenous solution a few formats have been established which make use of fluorescence resonance energy transfer (FRET). FRET happens when two fluorescent dyes are in close proximity to each other and the emission spectrum of the donor overlaps with the absorption spectrum of the acceptor.[8]

The spatially close arrangement of donor and acceptor is realized in TaqMan probes, which have a reporter dye covalently attached at the 5' end and a quencher at the 3' end.[9] For SNP genotyping a set of two TaqMan probes and two polymerase chain reaction (PCR) primers are used.[10] Both TaqMan probes only differ at the polymorphic site, with one probe complemantary to the wild type and one to the variant allele. After TaqMan probes have been hybridized to the polymorphic site in the annealing step of the PCR reaction, in the extension phase of the PCR reaction the 5' fluorophore is cleaved by the 5' nuclease activity of *Taq* polymerase. This enhances the fluorescence of the 5'donor which was formerly quenched due to the close proximity to the 3' acceptor in the TaqMan probe sequence. As a mismatched probe is not recognized by *Taq* Polymerase, the ratio of the fluorescence intensities of the two donor fluorophores in the TaqMan probes determine the genotype of the sample.

Another nucleic acid architecture where FRET plays an important role for detection are molecular beacons. Molecular beacons are stem-loop structures with a fluorescent reporter which is in close proximity to a universal quencher. When designed properly, the DNA target hybridizes with the loop portion of the molecular beacon and forces the stem to open, initiating a fluorescence signal from the emitter.[11] Specificity of the molecular beacons is achieved because mismatched probe-target duplexes dissociate at substantially lower temperatures than perfectly matched hybrids. For SNP genotyping usually a similar strategy like in the TaqMan approach is used. Two molecular beacons functionalized with different fluorescent reporters and exhibiting exact complementarities to the wild type and variant allele, respectively, are used in the same PCR reaction, which allows simultaneous determination of three allelic combinations.[12] Although these methods have proven their suitability for SNP detection and are commercially available, they have important limitations. First, they require site specific labeling of DNA with two extrinsic molecules, a donor and a quencher moiety. The dual labeling of DNA produces low yields and impurities may result in unfavorable effects on the sensitivity of the assay.[13] A second drawback is that although design algorithms for sequences are well elaborated, both probe sequences require significant optimization and redesign.

Instead of using interactions between two extrinsic fluorescent dyes, interactions of fluorophores with DNA bases can be used for specific detection of nucleic acid sequences. Flourescein labeled oligonucleotides have been used for mutation detection.[14] The inherent quenching of deoxyguanosine upon hybridization has been responsible for a decrease in fluorescence intensity. Also the opposite effect, hybridization induced dequenching of fluorescein labeled oligonucleotides, has been used for genotyping.[15] However, the omission of the second fluorophore - as used in probes exploiting the FRET effect described above - may lead to results which are difficult to predict. For example, in reference 14 it is stated that whether fluorescence intensity of the probe increases or decreases depends on the sequence. In fact, reference 14 reports de-quenching upon hybridization to a complementary sequence with a specific composition, viz. a sequence containing deoxyguanosine nucleotides. Besides the direction of the effect (quenching or de-quenching upon hybridization), which is sequence-specific, the extent of a de-quenching effect upon hybridization using probes without a second extrinsic fluorophore may pose a signal-to-noise problem.

Besides flourescein recently fluorene has been incorporated in the loop structure of a hairpin architecture.[16] A single base mismatch could be detected by de-quenching upon hybridization with the fully matched target and quenching upon

hybridization with a target containing a single base mutation. However, the hairpin structure necessary for the molecular beacon described in reference 16 imposes constraints on the sequence design of the molecular beacon. Furthermore, the fluorophore is directly attached to uridine such that interference with base pairing and stability of the duplex formed with a complementary sequence can not be excluded. Yet further, the fluorene-coupled nucleotide of reference 16 can only be incorporated using a oligonucleotide synthesizer. As known to the skilled person, the coupling efficiency for non-natural / derivatized nucleotides is generally bad.

In view of the limitations of probes and methods described in the prior art, the technical problem underlying the present invention was therefore the provision of improved and/or alternative means and methods for monitoring hybridization.

Accordingly, this invention relates to a compound having the general formula (I) ON1 - L1 - F - L2 - ON2 wherein ON1 and ON2 are independently selected from the group consisting of oligodeoxyribonucleotides, oligoribonucleotides or PNAs; L1 and L2 are linkers; and F is a fluorophore.

Both moieties ON1 and ON2 exhibit a backbone carrying a sequence of bases A, C, G and T, wherein T can also be U. Said sequence of bases renders the compounds of the invention suitable as probes which may hybridize to complementary sequences. Owing to the presence of two probe sequences provided by the moieties ON1 and ON2, the compounds of the present invention are also referred to as "twin probes" herein.

The term "linker" is well known in the art and relates to means for attaching a first moiety to a second moiety by introduction of a third moiety which is referred to as linker. Said attaching may involve two covalent bonds, one between the linker and the first moiety and a second between the linker and the second moiety. Alternatively, one or both linkages may be effected by non-covalent interactions.

The term "fluorophor" relates to molecules or portions of a molecule which are capable to emit light upon excitation. Emission preferably occurs at wavelengths between 100 and 1000 nm, more preferred between 300 and 600 nm. Excitation is effected by irradiation with light having wavelengths shorter as compared to emission wavelengths, preferably between 50 and 1000 nm, more preferred between 250 and 600 nm.

The term "PNA" stands for "peptide nucleic acid". In brief, a PNA is a synthetic DNA-mimic with an amide backbone in place of the sugar-phosphate backbone of DNA or RNA (Nielsen et al. (1991) Science, 254, 1497-1500 ; Egholm et al. (1993), Nature, 365, 566-568). PNAs exhibit several advantageous features. They are stable under acidic conditions and resistant to nucleases as well as proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060). Thus, PNA oligomers can be shorter than oligonucleotides when used as hybridisation probes. On the other hand, mismatches have a more destabilising effect, thus improving discrimination between perfect matches and mismatches. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to probe molecules.

Compounds wherein the ON moieties are chimeric molecules and/or derivatives of oligodeoxyribonucleotides, oligoribonucleotides or PNAs are also embraced by the present invention.

Chimeric molecules according to the invention include PNA chimera, DNA chimera and RNA chimera. PNA chimera according to the present invention are molecules comprising one or more PNA portions. The remainder of the chimeric molecule may comprise one or more DNA portions (PNA-DNA chimera), one or more RNA portions (PNA-RNA chimera), or one or more (poly)peptide portions (PNA-peptide chimera). DNA-peptide chimera according to the invention are molecules comprising one or more (poly)peptide portions and one or more DNA portions. The length of a portion of a chimeric molecule may range from 1 to n-1 bases, equivalents thereof or amino acids, wherein "n" is the total number of bases, equivalents thereof and amino acids of the entire molecule. Molecules comprising any combination of PNA, (poly)peptide, DNA and RNA portions are envisaged as well, provided said molecules exhibit at least one sequence of contiguous bases with a minimal length further specified below. The term "(poly)peptide" as used herein refers to a group of molecules which comprise the group of peptides, consisting of up to 30 amino acids, as well as the group of polypeptides, consisting of more than 30 amino acids.

The term "derivatives" relates to any of the above described molecules, wherein these molecules comprise one or more further groups or substituents different from PNA, (poly)peptides, DNA and RNA. All groups or substituents known in the art and used for the synthesis of these molecules, such as protection groups, and/or for applications involving these molecules, such as labels, (cleavable) linkers and functional groups for the attachment to other molecules or surfaces are envisaged.

There are at least two different ways by which a compound of the invention may hybridize with (a) matching sequence(s). First, and in case the sequences of both ON moieties are identical, two identical complementary sequences may each form a duplex with each one of the two ON moieties. Alternatively, a (longer) complementary sequence may comprise a sub-sequence complementary to ON1 and, separated by intervening bases, a sub-sequence complementary

to ON2. In such a case said complementary sequence may be a non-repetitive sequence and accordingly the sequences of ON1 and ON2 are different. Alternatively, said complementary sequence may comprise two or more repeating (inverted or non-inverted) sub-sequences two of which match ON1 and ON2 which in turn are identical.

The compounds of the invention advantageously undergo a significantly larger change in fluorescence properties than a compound having only one ON moiety when changing from an unhybridized state to a state where both ON1 and ON2 hybridize with (a) perfectly matching complementary sequence(s). This results from the enhanced (double) local concentration of duplexes in the proximity of the fluorophore as compared to compounds having only one ON moiety. The term "fluorescence properties" or briefly "fluorescence" include the wavelength of the emission maximum, the emission intensity at the emission maximum, the integral emission intensity, and the emission spectrum as whole.

Said change in fluorescence properties upon hybridization with (a) perfectly matching sequence(s) involves an increase in the integral emission intensity and/or the emission intensity at the emission maximum and/or a hypsochromic shift (blue shift) of the emission maximum, preferably both effects together. Whereas the applicant does not wish to be bound by any theory, this de-quenching upon hybridization is expected to result from a change in the extent of electron transfer reactions from the bases of the ON moieties to unoccupied electronic states of the central fluorophore which occur to a lesser extent when said bases are part of duplexes.

Reference Example 1 enclosed herewith provides a comparison of the properties of exemplary compounds analogous to those of the present invention but exhibiting only one ON moiety to the properties of compounds of the invention.

Surprisingly, the change in fluorescence properties upon hybridization with perfectly matching complementary sequences is yet larger than expected on the basis of the above described advantageous effect. While applicant does not wish to be bound by any theory, this larger than expected change in fluorescence properties may be attributed to the following effect. When not engaging in hybridization, the compounds of the invention perform $\pi$-stacking of the fluorene moieties, thereby forming supramolecular aggregates. This $\pi$-stacking entails quenching of fluorescence, i.e., a lower fluorescence intensity as compared to free form of the compound not involved in stacking. Upon hybridization, said $\pi$-stacking can not be maintained, and therefore, the change in fluorescence properties resulting from hybridization per se is accompanied by a de-quenching effect resulting from the breaking up of the supramolecular aggregates and the concomitant abolishment of $\pi$-stacking.

The central fluorophore of the compounds of the invention may be located between virtually any sequences of choice. In other words, and as opposed to molecular beacons and TaqMan probes known in the art, no intricate sequence design is necessary. The term "sequence design" refers to a careful selection process of probe sequences suitable for the respective assay, which frequently requires the skilled person to adopt a trial-and-error approach.

Compounds according to the invention differ advantageously from fluorescent probes known in the art carrying two fluorophors, i.e. a donor and an emitter, designed to exploit the FRET effect, such as TaqMan probes. By abolishing the need for dual labelling, fluorescent probes are provided by the present invention which can be obtained with better yields, less impurities and at lower cost. Furthermore, the synthesis of the twin probes of the invention does not suffer from the low coupling efficiency of non-natural or derivatized oligonucleotides in the course of oligonucleotide synthesis using an oligonucleotide synthesizer. For the synthesis of the twin probe of the invention, preferably standard nucleotides and commercially available linkers are used.

In a preferred embodiment, the fluorophore is selected from the group consisting of fluorenes, pyrenes, xanthenes including fluoresceines, rhodamines, coumarins, naphthylamines, acridines, benzoxazoles, benzodioxazoles, stilbenes, poly(p-phenylene vinylene)s, polythiophenes, poly(phenylene ethynylene)s and poly(paraphenylene)s. Naphthylamines may have the amino group in the alpha or beta position and include 1-dimethylaminonaphthyl-5-sulfonate, 1-anilino-8-naphthalene sulfonate and 2-p-toluidinyl-6-naphthalene sulfonate. Coumarins include 3-phenyl-7-isocyanatocoumarin ; acridines include 9-isothiocyanatoacridine and acridine orange; and benzoxazoles include N-(p-(2-benzoxazolyl)phenyl)maleimide.

It is understood that the use of the plural form, such as "fluorenes", indicates that not only fluorene itself, but also derivatives thereof known in the art at the priority date of the instant application are embraced.

Specific fluorophores according to the invention include the following commercially available (for example from Synthegen) fluorescent dyes: Tamra-dT, 5-Fluorescein (FITC), 5-Carboxyfluorescein (FAM), 6-Carboxyfluorescein (FAM), 3' 6-Carboxyfluorescein (FAM), 6-Carboxyfluorescein-DMT (FAM-X), 5(6)-Carboxyfluorescein (FAM), 6-Hexachlorofluorescein (HEX), 6-Tetrachlorofluorescein (TET), JOE, LightCycler Red 640, LightCycler Red 705, FAR-Fuchsia (5'-Amidite), FAR-Fuchsia (SE), FAR-Blue (5'-Amidite), FAR-Blue (SE), FAR-Green One (SE), FAR-Green Two (SE), Oregon Green 488, Oregon Green 500, Oregon Green 514, BODIPY FL-X, BODIPY FL, BODIPY-TMR-X, BODIPY R6G, BODIPY 650/665, BODIPY 564/570, BODIPY 581/591, BODIPY TR-X, BODIPY 630/650, BODIPY 493/503, Carboxyrhodamine 6G, MAX, 5(6)-Carboxytetramethylrhodamine (TAMRA), 6-Carboxytetramethylrhodamine (TAMRA), 5 (6)-Carboxy-X, Rhodamine (ROX), 6-Carboxy-X-Rhodamine (ROX), AMCA-X (Coumarin), Texas Red-X, Rhodamine Red-X, Marina Blue, Pacific Blue, Rhodamine Green-X, 7-diethylaminocoumarin-3-carboxylic acid, 7-methoxycoumarin-3-carboxylic acid, Cy3, Cy3B, Cy5, Cy5.5, DY-505, DY-550, DY-555, DY-610, DY-630, DY-633, DY-636, DY-650, DY-675, DY-676, DY-681, DY-700, DY-701, DY-730, DY-750, DY-751, DY-782, Cy3.5 and EDANS.

In a more preferred embodiment, the fluorophore is fluorene or 2,7-diphenyl-fluorene. Diphenyl-fluorene is an efficient blue emitter with a high fluorescence quantum efficiency, even in water.[17] Furthermore, fluorene is a building block of oligomeric and polymeric fluorophores. Such conjugated polymers are also within the scope of the present invention and are further detailed below. Conjugated polymers are known in the art as biosensors.[18-20]

In a yet more preferred embodiment, the fluorene moiety is 9-mono- or 9,9-di-substituted with a substituent selected from the group consisting of linear or branched alkyl groups having from 1 to 30 carbon atoms, preferably from 6 to 12 carbon atoms, aralkyl groups or oligoethylenglycol groups. Preferably, the oligoethylenglycol groups have from one to about ten ethylenglycol moieties, more preferred are tri- and hexa-ethylenglycol.

In a further preferred embodiment, the fluorophore is selected from the group of oligo- or poly-fluorenes, wherein one or more of the fluorene moieties may be 9-mono- or 9,9-di-substituted as described above, and wherein the terminal fluorenes may be substituted with a phenyl group at one or both of the free 2- and 7-positions, respectively.

Exemplary structures of oligo-fluorenes consisting of two and three fluorene moieties, respectively, and a general structure of poly-fluorenes are shown below. R may be either hydrogen or as defined above, wherein different occurrences of R may also refer to structurally distinct moieties.

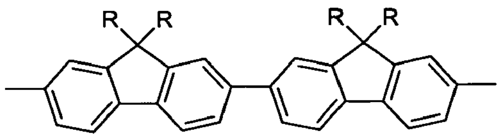

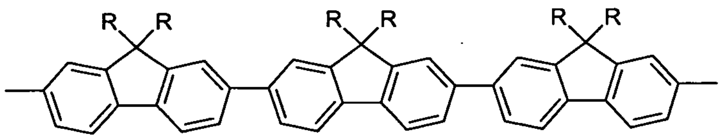

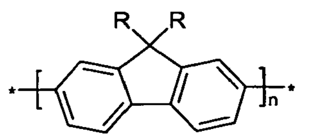

In a further preferred embodiment, L1 and L2 are such that the melting temperature of a first duplex formed by an ON moiety comprised in a compound according to the invention with its complementary sequence is not substantially altered as compared to the melting temperature of a second duplex formed by the corresponding free ON with its complementary sequence.

A not substantially altered melting temperature is indicative of a linker (i) which provides enough distance between the ON moiety and the fluorophore and (ii) which itself does not interfere significantly with duplex formation.

Melting of duplex DNA, be it a free duplex or a duplex formed by compound of the invention and a complementary sequence, can be easily monitored through changes in the ultraviolet spectrum with a UV-spectrophotometer. UV-spectra of double stranded oligonucleotide samples are recorded upon heating and cooling. Absorbance is plotted versus the temperature to give characteristic melting curves. Absorbance in the ultraviolet region increases as the double helix "melts" to two single strands. Reversibly, upon DNA duplex formation, the absorbance decreases. The hypochromicity effect (the reduction of absorbance of chromophores arranged in an ordered array) ranges in magnitude from 20-40% depending on duplex length. The structural changes in the strands that result from formation of Watson-Crick base pairs and associated interactions between neighboring bases (stacking interactions) cause the hypochromism. When duplex DNA unwinds (denatures), it is assumed that the changes in the extinction coefficient are proportional to the extent of denaturation. In other words, the change in absorbance at about 268 nm is directly proportional to the fraction of broken base pairs. The melting temperature $T_m$ is defined as the temperature at which 50% of a given oligonucleotide is hybridized

to its complementary strand.
It is furthermore preferred that the linkers do not exceed a length necessary for a change of the hybridization status of the ONs to exert a significant effect on the fluorescence properties of the fluorophore.

In a further preferred embodiment, the melting temperatures of said first duplex and said second duplex differ by less than 5 degrees on the Celsius scale.

In a further preferred embodiment, L1 and L2 are independently selected from the group consisting of amino-n-alkyl, mercapto-n-alkyl, amino-n-alkyl-X-alkyl, mercapto-n-alkyl-X-alkyl, wherein X is selected from the group consisting of O, S-S and $SO_2$ and wherein the alkyl groups independently from each other have from 1 to 30 carbon atoms, preferably 3, 6 or 12 carbon atoms; or oligoethylenglycols having from one to about ten ethylenglycol moieties, preferably tri- or hexa-ethylenglycol.
These and further suitable linkers are well known in the art and commercially available (see, for example, the catalogue from Glen Research, 22825 Davis Drive, Sterling, Virginia, 20164 USA). Further examples of linkers are the following: 5'-Amino-Modifiers (see e.g. B.A. Connolly and P. Rider, Nucleic Acids Res., 1985, 13, 4485; B.S. Sproat, B.S. Beijer, P. Rider, and P. Neuner, Nucleic Acids Res., 1987, 15, 4837; R. Zuckerman, D. Corey, and P. Shultz, Nucleic Acids Res., 1987, 15, 5305; P. Li, et al., Nucleic Acids Res., 1987, 15, 5275; G.B. Dreyer and P.B. Dervan, Proc. Natl. Acad. Sci. USA, 1985, 82, 968.); 5'-Thiol-Modifier C6 (see e.g. B.A. Connolly and P. Rider, Nucleic Acids Res., 1985, 13, 4485; B.S. Sproat, B.S. Beijer, P. Rider, and P. Neuner, Nucleic Acids Res., 1987, 15, 4837; R. Zuckerman, D. Corey, and P. Shultz, Nucleic Acids Res., 1987, 15, 5305; P. Li, et al., Nucleic Acids Res., 1987, 15, 5275.); 5'-Thiol-Modifier C6 S-S and Thiol Group at the 3'-Terminus (see e.g. B.A. Connolly and R. Rider, Nucleic Acids Res., 1985, 13, 4485; B.A. Connolly, Nucleic Acids Res., 1987, 15, 3131-3139; N.D. Sinha and R.M. Cook, Nucleic Acids Res., 1988, 16, 2659; A. Kumar, S. Advani, H. Dawar, and G.P. Talwar, Nucleic Acids Res., 1991, 19, 4561; R. Zuckermann, D. Corey, and P. Schultz, Nucleic Acids Res., 1987, 15, 5305; K.C. Gupta, P. Sharma, S. Sathyanarayana, and P. Kumar, Tetrahedron Lett., 1990, 31, 2471-2474; U. Asseline, E. Bonfils, R. Kurfurst, M. Chassignol, V. Roig, and N.T. Thuong, Tetrahedron, 1992, 48, 1233-1254; Gregg Morin, Geron Corporation, Personal Communication.); Spacer C3, Spacer C12, and dSpacer Phosphoramidites (see e.g. M. Durard, K. Chevrie, M. Chassignol, N.T. Thuong, and J.C. Maurizot, Nucleic Acids Res., 1990, 18, 6353; M. Salunkhe, T.F. Wu, and R.L. Letsinger, J. Amer. Chem. Soc., 1992, 114, 8768-8772; N.G. Dolinnaya, M. Blumenfeld, I.N. Merenkova, T.S. Oretskaya, N.F.Krynetskaya, M.G. Ivanovskaya, M. Vasseur, and Z.A. Shabarova, Nucleic Acids Res., 1993, 21, 5403-5407; M. Takeshita, C.N. Chang, F. Johnson, S. Will, and A.P. Grollman, J. Biol. Chem., 1987, 262, 10171-10179; M.W. Kalnik, C.N. Chang, A.P. Grollman, and D.J. Patel, Biochemistry, 1988, 27, 924-931.); 3'-Amino-Modifier C7 CPG (see e.g. J.G. Zendegui, K.M. Vasquez, J.H. Tinsley, D.J. Kessler, and M.E. Hogan, Nucleic Acids Res., 1992, 20, 307.); 3'-Amino Photolabile C6 CPG (see e.g. D.J. Yoo and M.M. Greenberg, *J. Org. Chem.,* 1995, 60, 3358-3364.; H. Venkatesan and M.M. Greenberg, *J. Org. Chem.*, 1996, 61, 525-529; D.L. McMinn and M.M. Greenberg, *Tetrahedron,* 1996, 52, 3827-3840.

In a more preferred embodiment, L1 and L2 are identical.

In a most preferred embodiment, the compounds of the invention have the structure

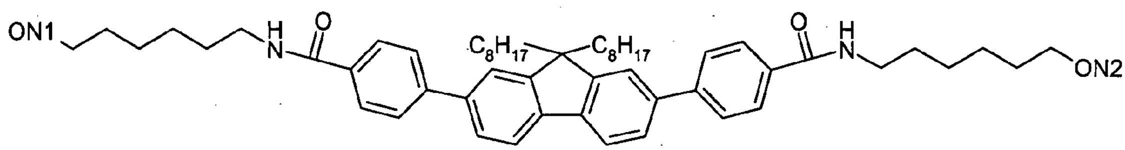

wherein $C_8H_{17}$ denotes n-octyl.
When a compound having the above structure hybridizes with a complementary sequence comprising two sub-sequences, one of which is complementary to ON1 and one of which is complementary to ON2, respectively, it is preferred that the length of the intervening stretch between said sub-sequences complementary to ON1 and ON2, respectively, is 8 bases.

In a further preferred embodiment, the lengths of the sequences of ON1 and independently of ON2 are at least 12, 15, 20 or 25 bases. Specifically envisaged lengths are 12, 13, 14, 15, 16, 17, 18, 19, 20, 21, 22, 23, 24 or 25 bases.

Preferably, the lengths of ON 1 and independently of ON2 do not exceed 100, 90, 80, 70, 60, 50, 45, 40, 35, 30, 29, 28, 27 or 26 bases.

Any combination of the above recited lengths, be it lower or upper limit, is specifically envisaged as lengths of the sequences of ON1 and ON2.

In a further preferred embodiment, ON1 and ON2 are connected to the linkers with different ends of their sequences. The term "different ends" of sequences in relation to distinct sequences implies that sequences of bases have a directionality. This is well known to the skilled person in case of oligodeoxyribonucleotides and oligoribonucleotides, wherein said different ends are the 5' and 3' end, respectively.

In case of PNAs the sugar-phosphate backbone of DNA and RNA, respectively, is replaced with a peptidic backbone made of N-(2-aminoethyl)-glycine building blocks. Accordingly, and instead of 5' and 3' ends, PNAs exhibit N-termini and C-termini.

In an alternative preferred embodiment, ON1 and ON2 are connected to the linkers with the same ends of their sequences.

In a more preferred embodiment, said same ends are the 5' ends in case of oligodeoxyribonucleotides and oligoribonucleotides or the N-termini in case of PNAs. Oligodeoxyribonucleotides and oligoribonucleotides with a 5' modification are easily accessible by automated synthesis. Furthermore, 5' modified oligodeoxyribonucleotides can be obtained from commercial sources.

In an alternative more preferred embodiment, said same ends are the 3' ends in case of oligodeoxyribonucleotides and oligoribonucleotides or the C-termini in case of PNAs.

In a yet more preferred embodiment of any one of the previous embodiments, the sequences of ON1 and ON2 are identical. This embodiment relates to a twin probe where two molecules of the same target polynucleotide comprising a sequence complementary to the sequence of ON1 (and at the same time of ON2) can bind to one compound of the invention. Thereby, the same hybridization event can occur twice in the proximity of the fluorophor. The inventors could demonstrate that saturation occurs at a twofold molar excess of the target polynucleotide.

The present invention also relates to a solid support carrying at least one compound according to the invention. The solid support according to the invention provides a surface for the attachment of the compounds of the invention. Said surface according to the invention may be any surface. The surface may be a coating applied to the support or carrier, or the surface of the support or carrier itself may be used. Support or carrier materials used in the art and comprising glass, plastic, gold and silicon are envisaged for the purpose of the present invention.

Attachment of the compounds of the invention to the surface of the solid support or carrier may be effected by any means known in the art. Attachment may involve the formation of one or more covalent bonds between the compound and the surface. Alternatively, it may involve no bond formation and be effected by non-covalent interactions. Attachment may involve a specifically coated or derivatized surface of the support. Alternatively, sufficient attachment may be provided by unspecific interaction between compound and surface. Attachment by also be effected by surface tension, i.e., by adhesion of liquid or solution comprising the compound to the surface.

In a preferred embodiment of the solid support, said solid support carries at distinct sites a plurality of compounds of the invention, wherein compounds at distinct sites may differ with regard to the ON sequence(s) and/or with regard to the fluorophore, and wherein preferably the distinct sites are arranged as array. It is particularly preferred that differen t ON sequence(s) are attached to different fluorophors.

The compounds of the invention may be delivered to any surface of the invention by a process called spotting. The term "spotting" designates the localized delivery of small amounts of liquid to a surface. Devices for spotting, including the simultaneous spotting of multiple samples, wherein said multiple samples, for example, have been picked up from one or more microtiter plates, are well known in the art. Delivery of material by spotting may involve a direct contact between the spotting pin and the surface. Alternatively, and similar to inkjet printers, such contact is not required. Preferably, spotting is done with a spotting robot comprising the spotting device and a computer controlling the function of the spotting device and providing a user interface. Amount of liquid and/or distance between spotting pins can be adjusted by the skilled artisan such that a surface with distinct sites is obtained. If arranged as array, the solid support of the invention is also referred to as array, microarray or chip.

Said different ON sequences may be designed such that any transcript and/or piece of genomic DNA from the genome of a cell or organism may be identified, preferably uniquely identified, by hybridization with its counterpart among the ON sequences. In view of the sensitivity of the twin probes of the invention to mismatches in the duplexes formed by the ON sequences with complementary sequences (for further details see below), the design of said solid support with a plurality of compounds of the invention attached thereto is extendible to the detection of mutations, SNPs and/or allelic variants. Such solid supports according to the invention are referred to as SNP arrays, SNP microarrays or SNP chips.

In a further preferred embodiment of said solid support, the compound(s) is/are covalently linked to the surface of said support via the free end of one of the ONs or, if present, via a substituent at the 9-position of fluorene.

The present invention also relates to a method of detecting a target polynucleotide in a sample comprising: (a) bringing into contact a compound according to the invention and a target polynucleotide under conditions allowing hybridization to occur; and (b) determining a change of the fluorescence of the fluorophore, wherein an increase in fluorescence intensity and/or a hypsochromic shift (blue shift) is indicative of the presence of a target polynucleotide comprising a sequence perfectly matching the ON sequence(s).

It is understood that the term "polynucleotide" embraces oligo- and polynucleotides. Also, it comprises DNA (polydeoxyribonucleotides), RNA (polyribonucleotides) and PNA (see further above for a definition of PNA). Furthermore, derivatives thereof and chimeric molecules comprising any combination of PNA, (poly)peptide, DNA and RNA portions are envisaged as well, provided said molecules exhibit at least one sequence of contiguous bases. The term "target" implies that said polynucleotide is a species the presence or absence of which in a sample is to be monitored.

Exemplary hybridization conditions suitable for the methods of the invention include 1 mM $MgCl_2$, and 20 mM Tris-HCl (pH 8.0) (see e.g. Tyagi S, Marras SAE, and Kramer FR (2000) Wavelength-shifting molecular beacons. Nat Biotechnol 18, 1191-1196), 1xPBS buffer (10x PBS 1 liter =80 g NaCl, 2g KCl, 26.8 g $Na_2HPO_4$-$7H_2O$ and 2.4 g $KH_2PO_4$ in 800 ml water, pH=7.4, volume 1 liter) (see e.g. Santangelo PJ, Nix B, Tsourkas A, and Bao G (2004) Dual FRET molecular beacons for mRNA detection in living cells. Nucleic Acids Res 32, e57) and 200mM Tris-HCl, pH 8.5, 75mM $MgCl_2$, 50mM KCl (see e.g. Gore HM, Wakeman CA, Hull RM, and McKillip JL (2003) Real-time molecular beacon NASBA reveals hblC expression from Bacillus spp. in milk. Biochem Biophys Res Commun 311, 386-390).
Said method may also be used for quantitation of the amount of target polynucleotide in a sample. For this purpose, the method may furthermore be calibrated by determining fluorescence for a dilution series with known concentrations of a polynucleotide comprising a sequence perfectly matching the ON sequence(s). Upon calibration, absolute amounts of target polynucleotide in a sample may be determined.

The present invention furthermore relates to a method of detecting one or more mutations or single nucleotide polymorphisms in a target polynucleotide in a sample comprising: (a) bringing into contact a compound according to the invention and a target polynucleotide under conditions allowing hybridization to occur; and (b) determining a change of the fluorescence of the fluorophore, wherein an increase in fluorescence intensity and/or a hypsochromic shift (blue shift) is indicative of the presence of a target polynucleotide comprising a sequence perfectly matching the ON sequence (s), and/or wherein a decrease in fluorescence intensity and/or a bathochromic shift (red shift) is indicative of the presence of a target polynucleotide comprising a sequence exhibiting at least one mutation or single nucleotide polymorphism.
The term "decrease" refers to a decrease in fluorescence intensity as compared to the fluorescence intensity of the twin probe in the hybridized state with a perfectly matching complementary sequence.
Detection of one particular mutant or one particular variant of a polymorphic sequence may be accomplished by either one of the following preferred embodiments of the method. A compound of the invention with perfectly matching ON sequence(s) is provided and the occurrence and optionally the extent of an increase in fluorescence intensity and/or a hypsochromic shift is observed. This approach is particularly suitable when the mutation or SNP is previously known. Alternatively, a compound of the invention with ON sequence(s) exhibiting a mismatch at the site of the mutation or SNP is provided and the occurrence and optionally the extent of a decrease in fluorescence intensity and/or a bathochromic shift is observed. This approach is particularly suitable when the mutation or SNP is not previously known. Both options are exemplified in Example 1 and by the corresponding data shown in Figure 1.
The term "nucleotide polymorphism" refers to the occurrence of one or more different nucleotides or bases at a given location on a chromosome. Polymorphisms are distinguished from mutations based on their prevalence. A threshold of 1% prevalence in a population of individuals is used herein for separating polymorphisms (more frequent) from mutations (less frequent). A single nucleotide polymorphism (SNP) is a polymorphism of a single nucleotide or base. The SNP database maintained at NCBI (http://www.ncbi.nlm.nih.gov/SNP/) divides SNPs into SNPs in the proximity of a known locus and such that are 5' further away than 2 kb from the most 5' feature of a gene and 3' further away than 500 bases from the most 3' feature of a gene. SNPs in the proximity of a known locus are further divided into SNPs occurring at an mRNA location and such that do not. SNPs occurring at an mRNA location comprise coding and non-coding SNPs.

The present invention furthermore relates to a method for the simultaneous detection of a plurality of alleles of a target polynucleotide in a sample comprising: (a) bringing into contact a plurality of compounds according to the invention and target polynucleotides under conditions allowing hybridization to occur, wherein said compounds differ with regard to both the fluorophore F and concomitantly with respect to the sequence(s) of the ONs, and wherein the ONs of a given compound perfectly match a given allele to be detected; and (b) determining a change of the fluorescence of each fluorophore, wherein an increase in fluorescence intensity and/or a hypsochromic shift (blue shift) is indicative of the presence of an allele of a target polynucleotide comprising a sequence perfectly matching the ON sequence(s) bound to the fluorophore the fluorescence of which is being measured, and/or wherein a decrease in fluorescence intensity and/or a bathochromic shift (red shift) is indicative of the presence of an allele of a target polynucleotide comprising a sequence exhibiting at least one mismatch.
The term "decrease" refers to a decrease in fluorescence intensity as compared to the fluorescence intensity of the twin probe in the hybridized state with a perfectly matching complementary sequence.
This is an extension of the above described method of detecting one or more mutations or single nucleotide polymorphisms in a target polynucleotide. It is particularly suitable when the presence of multiple variants of a given locus and/or transcript is known to occur or is expected.
Applied as a quantitative method, preferably upon calibration (see above), said method for the simultaneous detection of a plurality of alleles of a target polynucleotide provides information about the absolute and relative amount of different variant sequences or alleles, e.g. of one or more loci and/or corresponding transcripts.

In a preferred embodiment of any of the method of the invention, prior to step (a) or concomitantly with step (a) the target polynucleotide is amplified, preferably using a polymerase chain reaction (PCR).

PCR is well known in the art and is employed to make large numbers of copies of a target sequence. PCR comprises three major steps, which typically are repeated for 30 or 40 cycles. This is done on an automated cycler

device, which can heat and cool containers with the reaction mixture in a very short time. The first step is denaturation, e.g. at 94°C. During the denaturation, the double strand melts to form single stranded DNA. Concomitantly, all enzymatic reactions are stopped. These enzymatic reactions include, for example, the extension reaction of a previous PCR cycle. The second step is annealing, e.g. at 54°C. Bonds are constantly formed and broken between the single stranded primer and the single stranded template. The more stable bonds last a bit longer (primers that match exactly) and on the formed double stranded DNA comprising template and primer, the polymerase can attach and starts copying the template. Once a few bases are built in, the bonds are sufficiently strong between the template and the primer such that it does not break anymore. The third step is an extension step which is performed at a temperature of, for example, 72°C. This is the ideal temperature for polymerase activity. The primers, where there are a few bases built in, already exhibit a stronger binding to the template as compared to primers that are on positions with no exact match, which in turn get loose again (because of the higher temperature as compared to the annealing step) and do not give rise to an extension of the fragment.

In a more preferred embodiment, the target polynucleotide is amplified concomitantly with step (a) and the method is used to monitor and/or quantify amplification by determining the change of fluorescence intensity.

This embodiment relates to a real-time PCR method using the compounds of the invention instead of TaqMan probes known in the art and discussed herein above.

The above described methods may be performed as homogeneous assays. Alternatively, said compound(s) is/are attached to a solid support.

The present invention also relates to a method of synthesizing preferred compounds of the invention which comprises the steps of: (a) reacting 2,7-dibromo-fluorene or oligo-/poly-fluorene bromo-substituted at the free 2-position of one terminal fluorene moiety and the free 7-position of the other terminal fluorene moiety, optionally substituted at the 9-position(s) as defined herein above, respectively, with 4-carboxymethyl-phenyl-boronic acid; (b) converting the obtained bis-methylester into the bis-carboxylic acid; (c) activating the carboxylic acid groups; and (d) reacting the obtained activated ester with an ON carrying an amino linker, thereby obtaining said compound. This general method of synthesizing is further exemplified in Example 2 enclosed herewith.

The present invention also relates to a kit comprising (a) at least one of the compounds according to the invention; and (b) a manual with instructions for performing at least one of the methods of the invention directed to homogeneous assays.

The present invention furthermore provides a kit comprising (a) at least one of the solid supports according to the invention; and (b) a manual with instructions to perform the method of the invention using compounds of the invention attached to a solid support.

The Figures show:

**Figure 1:** Emission spectra of the twin probe **5** (conc. 1.7 x $10^{-7}$ mol) and corresponding duplexes of 5 with **ODN1** to **ODN4.** All oligonucleotides have been used in twofold excess in respect to **5.** (a) Non-hybridized twin probe **5** (b) Non-complementary **ODN2 + 5** (c) A to G mismatch **ODN4 + 5** (d) A to C mismatch **ODN3** + 5, (e) Complementary **ODN1 + 5.** Fluorescence spectra of hybridization buffer solutions containing 100 mM Tris, 100 mM NaCl, and 50 mM $MgCl_2$ were recorded at room temperature, using excitation wavelength of 350 nm. The sequences of probes **ODN1** to **ODN4** are given in Table 1 of Example 1. For the structures of compounds 1 to 5 please see Scheme 1 in Example 2.

**Figure 2:** Stern-Volmer plots measured in hybridization buffer for $G_{16}$, $C_{16}$, $T_{16}$, and $A_{16}$ with the mono-conjugate **4** (conc. 1.6 x $10^{-7}$ M). The emission spectra are measured upon excitation at 350 nm. On the x-axis, the concentration of the quencher ($G_{16}$, $C_{16}$, $T_{16}$, or $A_{16}$) in mM is shown. On the y-axis, the fluorescence intensity of the mono-conjugate without quencher ($I_0$) divided by the fluorescence intensity with quencher (I) is given.

The following examples illustrate the invention but should not be construed as being limiting.

### Example 1

Hybridization Experiments

To test the suitability of twin probe 5 for SNP detection, hybridization experiments have been carried out. Probe sequences used are shown in Table 1 below. Fluorescence emission spectra are shown in Figure 1.

**Table 1.** Results of the fluorescence measurements of the hybridization experiments and a list of the probe and target sequences.

| Identifier used herein | Sequence | Relative Fluorescence Intensity [%] | Wavelength of Emission Maximum [nm] |
|---|---|---|---|
| **5** | Probe : 5' $NH_2$-CTCAGATCTGGTCTAAC-3' | 11 | 432 |
| **ODN1** | Comp : 3'-GAGTCTAGACCAGATTG-5' | 100 | 417 |
| **ODN2** | Non-Comp : 3'-ACTCTTATCACATACGC-5' | 18 | 439 |
| **ODN3** | A to C : 3'-GAGTCCAGACCAGATTG-5' | 49 | 420 |
| **ODN4** | A to G : 3'-GAGTCGAGACCAGATTG-5' | 25 | 420 |

The non-hybridized twin probe **5** shows relatively low fluorescence intensity in buffer solution (100 mM Tris, 100 mM NaCl, and 50 mM $MgCl_2$ at 25°C) upon excitation with 350 nm. Hybridization of twin probe **5** with the target sequence **ODN1** that fully matches the probe sequence leads to a ten-fold increase in fluorescence intensity. Simultaneously, a blue shift of the emission band occurs from 432 to 417 nm. When **5** is incubated with a non-complementary target **ODN2** only a small fluorescence enhancement is observed accompanied by a red-shift of the emission maxima from 432 to 439 nm. On the basis of the hybridization results of the twin probe **5** with the fully matching and a non-complementary sequence, the possibility of detecting single nucleotide mismatches has been checked. Therefore, two target sequences **ODN3** and **ODN4** containing an A to C and an A to G mismatch, respectively, were examined. Upon hybridization, the mismatch is located within the helix of probe and target sequences. The mismatched bases are marked as underlined letters in Table 1. When the probe **5** is hybridized with the mismatched sequences **ODN3** and **ODN4** the emission intensities drop to 49% and 25%, respectively, compared to the signal achieved with the fully matched sequence **ODN1.** Upon duplex formation of the mismatched sequences **ODN3** and **ODN4** with the probe 5, the emission maxima are shifted hypsochromically from 432 to 420 nm (Figure 1, Table 1). The fluorescence measurements, which are summarized in Table 1, clearly demonstrate that the twin probe according to the present invention is well suited for sequence specific DNA assays and even single base mismatches can be detected with high accuracy.

**Example 2**

Twin Probe Synthesis

The synthesis of the fluorescent core molecule starts from fluorene derivative **1** (see Scheme1 below). 2,7-Dibromo-9,9-di-n-octylfluorene **(1)** is reacted in a palladium catalyzed Suzuki coupling[21] with 4-carboxy-methylphenylboronic acid. The bis-methylester is converted into the bis-carboxylic acid **2** by KOH. Subsequent activation of the carboxylic acid groups is carried out with N-hydroxysuccinimide and dicyclohexylcarbodiimide. The corresponding active ester **3** is reacted with a 17mer oligonucleotide sequence (3'-CAATCTGGTCTAGACTC-5') carrying a 5' amino linker to form the mono conjugate **4** as a byproduct and the desired twin probe **5.**

**Scheme 1:** Synthesis of Oligonucleotide conjugates [a)]

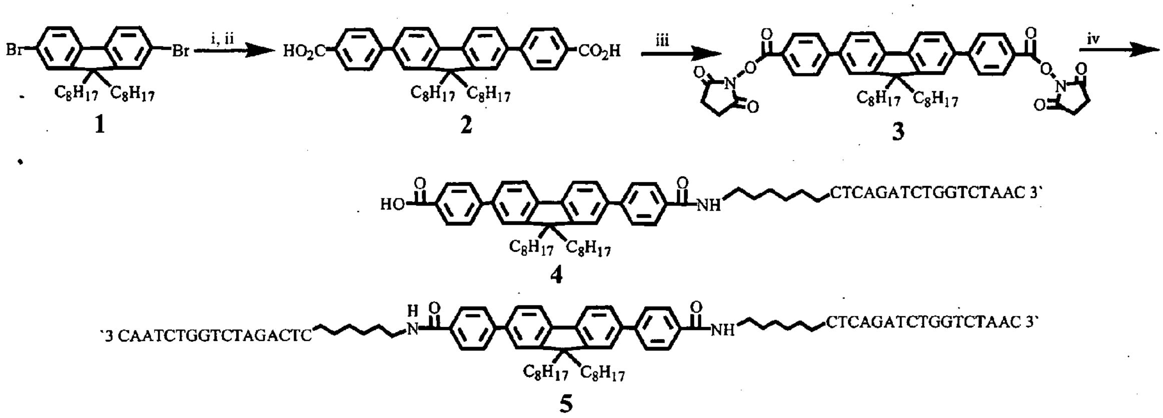


1 2 3 4 5

a) Reagents and conditions: (i) 4-carboxymethylphenylboronicacid, $K_2CO_3$, THF/$H_2O$, $Pd(PPh_3)_4$, 80°C 16 h, 73%; ii) KOH, EtOH/$H_2O$, reflux, 3 h, 88%; (iii) N-Hydroxysuccinimide (NHS), dicyclehexylcarbodiimide (DCC), DMF, room temperature, overnight; (iv) 5'-amino modified oligonucleotide, DMF/sodiumtetraborate decahydrate, room temperature, overnight.

**Reference Example 1**

Hybridization Experiments

Hybridization experiments have also been carried out with the fluorene derivative **4,** connected only to one oligonucleotide. This compound exhibits high fluorescence intensity in buffer solution. Upon hybridization with its complementary sequence a decrease in fluorescence intensity is detected (data not shown). For the purpose of getting more insight into the factors that determine the fluorescence behavior of the central fluorophore within the twin probe **5,** the mono-conjugate **4** is a very well suited model compound for comparative studies. To investigate the effect of the four different nucleotides on the optical properties of the fluorene moiety, quenching experiments have been performed with the mono conjugate **4** and $A_{16}$, $G_{16}$, $C_{16}$, $T_{16}$, respectively. **4** has been utilized in the quenching experiments because in contrast to **2** it is soluble in buffer solutions. The fluorescence intensities of the mono-conjugate **4** (conc. 1.6 x $10^{-7}$ M) in the presence of various concentrations ranging from 7.0 x $10^{-5}$ to 1.5 x $10^{-3}$ M of the four different 16mer oligonucleotides have been measured and expressed in typical Stern-Volmer plots[1].[22] With increasing concentration of all four oligonucleotides a decrease of the emission intensity of **4** is observed. An exponential relationship in the Stern-Volmer plot is obtained for quencher concentrations higher than 5.0 x $10^{-4}$ M (data not shown), which can be explained by sphere-of-action quenching also present in other polyelectrolyte systems.[23] For oligonucleotide concentrations below 5.0 x $10^{-4}$ M a linear dependence in the Stern-Volmer plot (Figure 2) is obtained from which $K_{SV}$ values can be calculated. The most efficient quencher is $G_{16}$ with a $K_{SV}$ of 5.1 x $10^{-4}$ $M^{-1}$. The next highest $K_{SV}$ are measured for $C_{16}$ and $T_{16}$ corresponding to 3.8 x $10^{-4}$ $M^{-1}$ and 3.5 x $10^{-4}$ $M^{-1}$, respectively. The difference between $C_{16}$ and $T_{16}$ falls within the experimental uncertainty of the measurement. The oligonucleotide with the lowest effect on the emission intensity of the fluorene moiety is $A_{16}$ with a $K_{SV}$ of 1.3 x $10^{-4}$ $M^{-1}$. The data of the quenching experiments are also in agreement with the results obtained from hybridization. An A to G mismatch in ODN4 leads to a higher extent of quenching than the A to C mismatch in ODN3 underpinning the fact that G is the most efficient quencher for fluorene connected to oligonucleotides in conjugates **4** and **5.** Guanosine residues have also been reported to quench the emission of other fluorescent dyes efficiently.[14, 24]

[1] Stern-Vollmer plots show the influence of a quencher on an emitter. The Stern-Vollmer equation reads

$$I_0/I = 1 + K_{sv}\,[Q],$$

wherein $I_0$ is the fluorescence intensity without quencher,

$I_0$ is the fluorescence intensity with quencher,
$K_{SV}$ is the Stern-Vollmer constant, and

[Q] is the concentration of the quencher.

The Stern-Vollmer constant is a molecule-specific quantity and determines how efficient a quencher reduces the fluorescence intensity of a fluorophore.

**Further References**

[1] http://genome.ucsc.edu.
[2] http://www.ensembl.org.
[3] http://www.ncbi.nlm.nih.gov.
[4] R. Sachidanandam, D. Weissman, S. C. Schmidt, J. M. Kakol, L. D. Stein, G. Marth, S. Sherry, J. C. Mullikin, B. J. Mortimore, D. L. Willey, S. E. Hunt, C. G. Cole, P. C. Coggill, C. M. Rice, Z. M. Ning, J. Rogers, D. R. Bentley, P. Y. Kwok, E. R. Mardis, R. T. Yeh, B. Schultz, L. Cook, R. Davenport, M. Dante, L. Fulton, L. Hillier, R. H. Waterston, J. D. McPherson, B. Gilman, S. Schaffner, W. J. Van Etten, D. Reich, J. Higgins, M. J. Daly, B. Blumenstiel, J. Baldwin, N. S. Stange-Thomann, M. C. Zody, L. Linton, E. S. Lander, D. Altshuler, *Nature* **2001,** *409,* 928.
[5] http://www.ornl.gov/sci/techresources/Human_Genome/project/journals/insights.html.
[6] M. M. Shi, *Clinical Chemistry* **2001,** 47, 164.
[7] P.-Y. Kwok, *Annu. Rev. Genomics Hum. Genet.* **2001,** 2, 235.
[8] R. M. Clegg, *Methods Enzymol.* **1992,** 211, 353.
[9] K. J. Livak, S. J. A. Flood, J. Marmaro, W. Giusti, K. Deetz, *PCR Methods Appl.* **1995,** 4, 357.
[10] M. M. Shi, S. P. Myrand, M. R. Bleavins, F. A. Igiesia, *J. Clin. Pathol.: Mol. Pathol.* **1999,** 52, 295.
[11] G. Bonnet, S. Tyagi, A. Libchaber, F. R. Kramer, *Proc. Natl. Acad. Sci. USA.* **1999,** 96, 6171.
[12] S. A. E. Marras, F. R. Kramer, S. Tyagi, *Genet. Anal.* **1999,** *14*, 151.
[13] J. P. Knemeyer, N. Marme, M. Sauer, *Anal. Chem.* **2000,** *72*, 3717.
[14] A. O. Crockett, C. T. Wittwer, *Anal. Biochem.* **2001,** *290*, 89.
[15] C. P. Vaughn, K. S. J. E. Johnson, *Am. J. Pathol.* **2003,** *1*, 29.
[16] G. T. Hwang, Y. J. Seo, B. H. Kim, *J. Am. Chem.* Soc. **2004,** *126,* 6528.
[17] M. Stork, B. S. Gaylord, A. J. Heeger, G. C. Bazan, *Adv. Mat.* **2002,** *14,* 361.
[18] L. H. Chen, D. W. McBranch, H. L. Wang, R. Helgeson, F. Wudl, D. G. Whitten, *Proceedings of the National Academy of Sciences of the United States of America* **1999,** *96*, 12287.
[19] D. L. Wang, X. Gong, P. S. Heeger, F. Rininsland, G. C. Bazan, A. J. Heeger, *Proceedings of the National Academy of Sciences of the United States of America* **2002,** 99, 49.
[20] H. A. Ho, M. Boissinot, M. G. Bergeron, G. Corbeil, K. Dore, D. Boudreau, M. Leclerc, *Angewandte Chemie-International Edition* **2002,** 41, 1548.
[21] N. Miyaura, A. Suzuki, *Chem. Rev.* **1995,** 95, 2457.
[22] B. Valeur, *Molecular Fluorescence: Principles and Applications,* Wiley-VCH Verlag GmbH, Weinheim, **2001.**
[23] J. Wang, D. Wang, E. K. Miller, D. Moses, G. C. Bazan, A. J. Heeger, *Macromol.* **2000,** *33,* 5153.
[24] T. Heinlein, J. P. Knemeyer, O. Piestert, M. Sauer, *J. Phys. Chem. B* **2003,** *107*, 7957.


**Claims**

**1.** A compound having the general formula (I)

ON1-L1-F-L2-ON2

wherein
ON1 and ON2 are independently selected from the group consisting of oligodeoxyribonucleotides, oligoribonucleotides or PNAs;
L1 and L2 are linkers; and
F is a fluorophore.

**2.** The compound according to claim 1, wherein the fluorophore is selected from the group consisting of fluorenes, pyrenes, xanthenes including fluoresceines, rhodamines, coumarins, naphthylamines, acridines, benzodioxazoles, stilbenes, poly(p-phenylene vinylene)s, polythiophenes, poly(phenylene ethynylene)s and poly(para-phenylene)s.

**3.** The compound according to claim 1 or 2, wherein the fluorophore is fluorene or 2,7-diphenyl-fluorene.

**4.** The compound according to claim 3, wherein the fluorene moiety is 9-monoor 9,9-di-substituted with a substituent selected from the group consisting of linear or branched alkyl groups having from 1 to 30 carbon atoms, preferably from 6 to 12 carbon atoms, aralkyl groups or oligoethylenglycol groups.

**5.** The compound according to claim 1, wherein the fluorophore is selected from the group of oligo- or poly-fluorenes, wherein one or more of the fluorene moieties may be 9-mono- or 9,9-di-substituted as defined in claim 4, and wherein the terminal fluorenes may be substituted with a phenyl group at one or both of the free 2- and 7-positions, respectively.

**6.** The compound according to any of the preceding claims, wherein L1 and L2 are such that the melting temperature of a first duplex formed by an ON comprised in a compound according to any of the preceding claims with its complementary sequence is not substantially altered as compared to the melting temperature of a second duplex formed by the corresponding free ON with its complementary sequence.

**7.** The compound according to claim 6, wherein the melting temperatures of said first duplex and said second duplex differ by less than 5 degrees on the Celsius scale.

**8.** The compound according to any of the preceding claims, wherein L1 and L2 are independently selected from the group consisting of amino-n-alkyl, mercapto-n-alkyl, amino-n-alkyl-X-alkyl, mercapto-n-alkyl-X-alkyl, wherein X is selected from the group consisting of O, S-S and $SO_2$ and wherein the alkyl groups independently from each other have from 1 to 30 carbon atoms, preferably 3, 6 or 12 carbon atoms; or oligoethylenglycols, preferably tri- or hexa-ethylenglycol.

**9.** The compound according to any of the preceding claims, wherein L1 and L2 are identical.

**10.** The compound of claim 1 having the structure

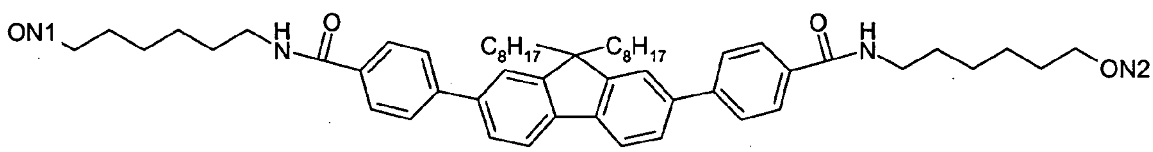

**11.** The compound according to any of the preceding claims, wherein the lengths of the sequences of ON1 and independently of ON2 are at least 12, 15, 20 or 25 bases.

**12.** The compound according to any one of claims 1 to 11, wherein ON 1 and ON2 are connected to the linkers with different ends of their sequences.

**13.** The compound according to any one of claims 1 to 11, wherein ON 1 and ON2 are connected to the linkers with the same ends of their sequences.

**14.** The compound of claim 13, wherein said same ends are the 5' ends in case of oligodeoxyribonucleotides and oligoribonucleotides or the N-termini in case of PNAs.

**15.** The compound of claim 13, wherein said same ends are the 3' ends in case of oligodeoxyribonucleotides and oligoribonucleotides or the C-termini in case of PNAs.

**16.** The compound according to any of the preceding claims, wherein the sequences of ON 1 and ON2 are identical.

**17.** A solid support carrying at least one compound according to any one of the preceding claims.

**18.** The solid support of claim 17 carrying at distinct sites a plurality of compounds according to any one of the preceding claims, wherein compounds at distinct sites may differ with regard to the ON sequence(s) and/or with regard to the fluorophore, and wherein preferably the distinct sites are arranged as array.

**19.** The solid support according to claim 17 or 18, wherein the compound(s) is/are covalently linked to the surface of said support via the free end of one of the ONs or, if present, via a substituent at the 9-position of fluorene.

**20.** A method of detecting a target polynucleotide in a sample comprising:

(a) bringing into contact a compound according to any one of the preceding claims and a target polynucleotide under conditions allowing hybridization to occur; and
(b) determining a change of the fluorescence of the fluorophore, wherein an increase in fluorescence intensity and/or a hypsochromic shift (blue shift) is indicative of the presence of a target polynucleotide comprising a sequence perfectly matching the ON sequence(s).

**21.** A method of detecting one or more mutations or single nucleotide polymorphisms in a target polynucleotide in a sample comprising:

(a) bringing into contact a compound according to any one of the preceding claims and a target polynucleotide under conditions allowing hybridization to occur; and
(b) determining a change of the fluorescence of the fluorophore, wherein an increase in fluorescence intensity and/or a hypsochromic shift (blue shift) is indicative of the presence of a target polynucleotide comprising a sequence perfectly matching the ON sequence(s), and/or wherein a decrease in fluorescence intensity and/or a bathochromic shift (red shift) is indicative of the presence of a target polynucleotide comprising a sequence exhibiting at least one mutation or single nucleotide polymorphism.

**22.** A method for the simultaneous detection of a plurality of alleles of a target polynucleotide in a sample comprising:

(a) bringing into contact a plurality of compounds according to any one of the preceding claims and target polynucleotides under conditions allowing hybridization to occur, wherein said compounds differ with regard to both the fluorophore F and concomitantly with respect to the sequence(s) of the ONs, and wherein the ONs of a given compound perfectly match a given allele to be detected; and
(b) determining a change of the fluorescence of each fluorophore, wherein an increase in fluorescence intensity and/or a hypsochromic shift (blue shift) is indicative of the presence of an allele of a target polynucleotide comprising a sequence perfectly matching the ON sequence(s) bound to the fluorophore the fluorescence of which is being measured, and/or wherein a decrease in fluorescence intensity and/or a bathochromic shift (red shift) is indicative of the presence of an allele of a target polynucleotide comprising a sequence exhibiting at least one mismatch.

**23.** The method according to any one of claims 20 to 22, wherein prior to step (a) or concomitantly with step (a) the target polynucleotide is amplified, preferably using a polymerase chain reaction (PCR).

**24.** The method of claim 23, wherein the target polynucleotide is amplified concomitantly with step (a) and the method is used to monitor and/or quantify amplification by determining the change of fluorescence intensity.

**25.** The method according to any one of claims 20 to 24, wherein said compound(s) is/are attached to a solid support.

**26.** A method of synthesizing a compound according to claim 3 to 5 or 10 comprising the steps of:

(a) reacting 2,7-dibromo-fluorene or oligo-/poly-fluorene bromo-substituted at the free 2-position of one terminal fluorene moiety and the free 7-position of the other terminal fluorene moiety, optionally substituted as defined in claim 4 or 5, respectively, with 4-carboxy-methyl-phenylboronic acid;
(b) converting the obtained bis-methylester into the bis-carboxylic acid;
(c) activating the carboxylic acid groups; and
(d) reacting the obtained activated ester with an ON carrying an amino linker, thereby obtaining said compound.

**27.** A kit comprising

(a) at least one of the compounds of claims 1 to 16; and
(b) a manual with instructions for performing at least one of the methods of claims 20 to 24.

**28.** A kit comprising

(a) at least one of the solid supports of claims 17 to 19; and
(b) a manual with instructions to perform the method according to claim 25.

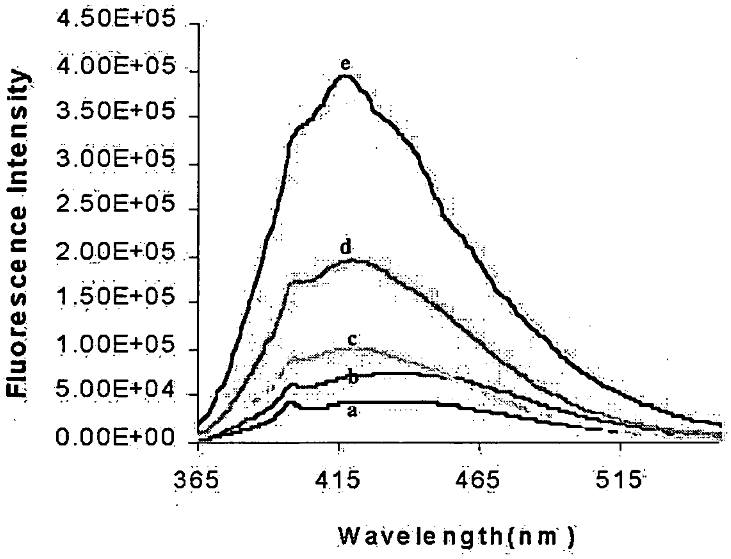


Figure 1

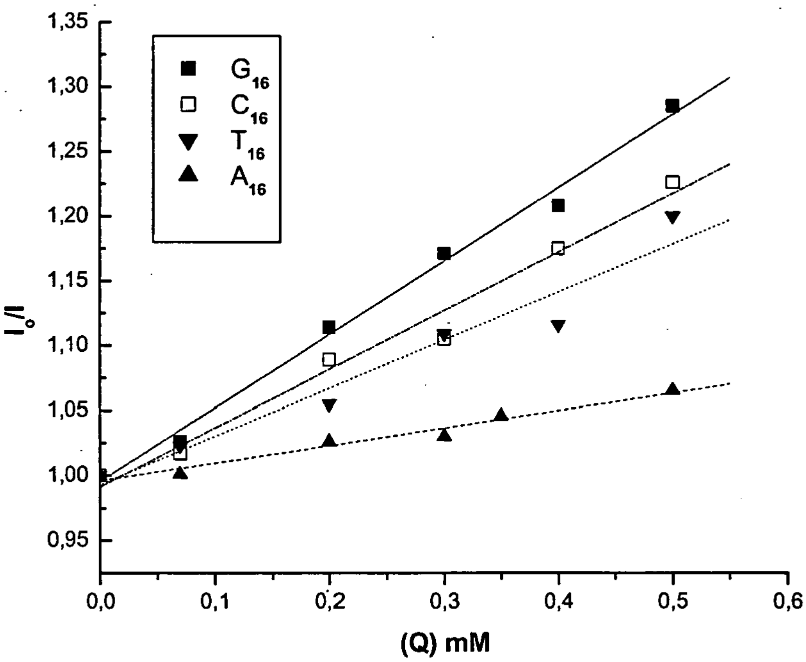


Figure 2

European Patent Office

# EUROPEAN SEARCH REPORT

Application Number
EP 04 03 0341

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 02/072865 A (BOSTON PROBES, INC) 19 September 2002 (2002-09-19) * page 21, line 18 - line 31; claims 1-144 * | 1-28 | C12Q1/68 C07H21/00 |
| A | GAYLORD BRENT S ET AL: "DNA hybridization detection with water-soluble conjugated polymers and chromophore-labeled single-stranded DNA." JOURNAL OF THE AMERICAN CHEMICAL SOCIETY. 29 JAN 2003, vol. 125, no. 4, 29 January 2003 (2003-01-29), pages 896-900, XP002317891 ISSN: 0002-7863 * the whole document * | 1-28 | |
| D,A | CROCKETT ANDREW O ET AL: "Fluorescein-labeled oligonucleotides for real-time PCR: Using the inherent quenching of deoxyguanosine nucleotides" ANALYTICAL BIOCHEMISTRY, ACADEMIC PRESS, SAN DIEGO, CA, US, vol. 290, no. 1, 1 March 2001 (2001-03-01), pages 89-97, XP002200212 ISSN: 0003-2697 * the whole document * | 1-28 | |

-/--

TECHNICAL FIELDS SEARCHED (Int.Cl.7)
C12Q
C07H

The present search report has been drawn up for all claims

| Place of search | Date of completion of the search | Examiner |
|---|---|---|
| Munich | 16 February 2005 | Bradbrook, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

European Patent Office

# EUROPEAN SEARCH REPORT

Application Number
EP 04 03 0341

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | VAUGHN CECILY P ET AL: "Hybridization-induced dequenching of fluorescein-labeled oligonucleotides: A novel strategy for PCR detection and genotyping." AMERICAN JOURNAL OF PATHOLOGY, vol. 163, no. 1, July 2003 (2003-07), pages 29-35, XP002317892 ISSN: 0002-9440 * the whole document * ----- | 1-28 | |
| A | BERNIUS M ET AL: "Light-emitting diodes based on fluorene polymers" THIN SOLID FILMS, ELSEVIER-SEQUOIA S.A. LAUSANNE, CH, vol. 363, no. 1-2, March 2000 (2000-03), pages 55-57, XP004189266 ISSN: 0040-6090 * page 55, column 1, paragraph 1 - page 56, column 1, paragraph 1 * ----- | 1-16 | |

TECHNICAL FIELDS SEARCHED (Int.Cl.7)

The present search report has been drawn up for all claims

2

| Place of search | Date of completion of the search | Examiner |
|---|---|---|
| Munich | 16 February 2005 | Bradbrook, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 04 03 0341

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-2005

| Patent document cited in search report | Publication date | Patent family member(s) | Publication date |
|---|---|---|---|
| WO 02072865 A | 19-09-2002 | CA 2439750 A1 | 19-09-2002 |
| | | EP 1412517 A2 | 28-04-2004 |
| | | JP 2004524032 T | 12-08-2004 |
| | | WO 02072865 A2 | 19-09-2002 |
| | | US 2003077608 A1 | 24-04-2003 |

EPO FORM P0459

For more details about this annex : see Official Journal of the European Patent Office, No. 12/82